# EUROPEAN PATENT APPLICATION

(11) **EP 4 684 735 A1**
(43) Date of publication of application: **28.01.2026**
(21) Application number: 24190091.9
(22) Date of filing: 22.07.2024
(51) Int. Cl.: A61B 6/42, A61B 6/00

(54) **METHOD FOR PAIRING A MOBILE X-RAY DETECTOR WITH AN X-RAY TUBE SYSTEM**

(71) Applicant: Koninklijke Philips N.V., 5656 AG Eindhoven (NL)
(72) Inventor: GOOßEN, Andre, Eindhoven (NL); BYSTROV, Daniel, Eindhoven (NL); KROENKE-HILLE, Sven, 5656AG Eindhoven (NL); BRUECK, Heiner Matthias, Eindhoven (NL); VON BERG, Jens, Eindhoven (NL); PAUL, Soubhik, Eindhoven (NL); YOUNG, Stewart Matthew, Eindhoven (NL)
(74) Representative: Philips Intellectual Property & Standards

(57) **Abstract**

The invention relates to a method (10) for pairing a mobile X-ray detector (2) with an X-ray tube system (1), wherein the mobile X-ray detector (2) comprises a first wireless communication interface and the X-ray tube system (1) comprises an X-ray tube (5), at least one sensor (6), a computing unit and a second wireless communication interface. According to the method (10), sensor data is obtained (SI) by the at least one sensor (6) and transmitted (S2) to the computing unit. Based on the received sensor data, a mobile X-ray detector (2) located in a predefined part of space is identified (S3) by the computing unit. Then, the mobile X-ray detector (2) is paired with the X-ray tube system (1) by the computing unit, via the second wireless communication interface, and via the first wireless communication interface. The invention further relates to a corresponding X-ray tube system (1).

## Description

### FIELD OF THE INVENTION

The invention relates to medical X-ray systems, in particular to X-ray systems that can be used with a mobile X-ray detector, more particularly to a method for pairing a mobile X-ray detector with an X-ray tube system. Further, the invention relates to a corresponding X-ray tube system.

### BACKGROUND OF THE INVENTION

Portable X-ray detectors have replaced computed radiography (CR) cassettes in a digital workflow and are just as easy to handle. Clinics often use and share multiple portable detectors in different sizes, sometimes different models, between X-ray rooms or systems. After pairing, the portable detector supports an intuitive workflow as it is easy to position and less bulky than any built-in detector. However, the pairing of portable detectors to the X-ray system, i.e., the identifying mobile detector and X-ray machine to each other, is rather bulky. For this purpose, users have to scan a barcode in the control room in order to register the detector with the system. As a consequence, the operator has to decide upfront which detector to use and upon failed pairing or if the operator changes mind, the detector has to be registered in the control room again, hence wasting a lot of time.

### SUMMARY OF THE INVENTION

It is therefore an object of the present invention to provide an improved method for pairing a mobile X-ray detector with an X-ray tube system, in particular a method that avoids the pairing operations described above. Further, it is an object of the present invention to provide a corresponding X-ray tube system.

The object of the present invention is solved by the subject matter of the independent claims, wherein further embodiments are incorporated in the dependent claims.

In an aspect of the present invention, a method for pairing a mobile X-ray detector with an X-ray tube system is provided.

In this context, a mobile X-ray detector is an X-ray detector that can be moved, in particular between different rooms. The mobile X-ray detector may be a portable X-ray detector, i.e., a mobile X-ray detector that can be carried, but may also be a mobile X-ray detector attached to a movable detector stand.

The X-ray tube system is a medical system comprising an X-ray tube. The X-ray tube system may comprise a dedicated X-ray detector. In this case, the X-ray tube system may be referred to as X-ray system. Alternatively, the X-ray tube system may have no dedicated X-ray detector, i.e., it is to be operated solely with mobile X-ray detectors. In particular, the mobile X-ray detector and the X-ray tube system may form a digital X-ray system. Further, the X-ray tube system may be configured to support multiple mobile X-ray detectors, more particularly via detector sharing.

Pairing of the mobile X-ray detector with the X-ray tube system may mean identifying the mobile X-ray detector with the X-ray tube system. In particular, pairing of the mobile X-ray detector with the X-ray tube system may comprise setting up the X-ray tube system and/or the mobile X-ray detector such that X-ray images may be obtained with the X-ray tube system using the mobile X-ray detector. More particularly, pairing of the mobile X-ray detector with the X-ray tube system may comprise setting up a communication between the X-ray tube system and the mobile X-ray detector, configuring the X-ray tube system to acquire one or more X-ray images with the mobile X-ray detector, and/or configuring the mobile X-ray detector to acquire one or more X-ray images with the X-ray tube system.

The mobile X-ray detector comprises a first wireless communication interface. Said first wireless communication interface may be used for pairing the mobile X-ray detector with the X-ray tube system, for communication between the mobile X-ray detector and the X-ray tube system, and/or for transmitting X-ray images obtained by the mobile X-ray detector.

The X-ray tube system comprises an X-ray tube. Said X-ray tube is configured to emit X-rays that can be detected by an X-ray detector, in particular by the mobile X-ray detector, to obtain an X-ray image, in particular a medical X-ray image.

Further, the X-ray tube system comprises at least one sensor, a computing unit and a second wireless communication interface. The second wireless communication interface may be used for pairing the X-ray tube system with a mobile X-ray detector and for communication between the X-ray tube system and the mobile X-ray detector.

According to the method, sensor data is obtained by the at least one sensor. Said sensor data is then transmitted by the at least one sensor to the computing unit. Accordingly, the sensor data is received by the computing unit from the at least one sensor. Transmitting and receiving the sensor data may be performed using a wired connection and/or a wireless connection. Further, the sensor data may be obtained, transmitted, and received periodically, e.g., once per second, or several times per second. The sensor data may be raw sensor data obtained by the at least one sensor or may be pre-processed by the at least one sensor.

Based on the received sensor data, the computing unit identifies a mobile X-ray detector located in a predefined part of space. That is, the computing unit may process and/or analyze the received sensor data to identify the mobile X-ray detector located in the predefined part of space.

Finally, the computing unit pairs, via the second wireless communication interface, and via the first wireless communication interface, the mobile X-ray detector with the X-ray tube system. In this context, pairing may comprise checking whether a mobile X-ray detector has already been paired with the X-ray tube system. If no mobile X-ray detector has been paired with the X-ray tube system, the newly identified mobile X-ray detector will be paired with the X-ray tube system. If the identified mobile X-ray detector is already paired with the X-ray tube system, the pairing will be maintained. And if a different mobile X-ray detector is paired with the X-ray tube system, this pairing will be terminated, and the newly identified X-ray detector will be paired with the X-ray tube system.

This automatic pairing of the mobile X-ray detector with the X-ray tube system significantly improves the workflow of pairing the mobile X-ray detector with the X-ray tube system. In particular, no extra manual interaction is necessary for the pairing, saving time for the staff operating the X-ray system. More particularly, the workflow for operating the X-ray tube system with the mobile X-ray detector may be rendered more natural and fluent. Also, changing the mobile X-ray detector paired with the X-ray tube system is simple and fast with the described method. Moreover, no dedicated extra piece of hardware is necessary to perform the pairing of the mobile X-ray detector with the X-ray tube system. Also, the described method provides increased reliability, since pairing the X-ray tube system with a wrong mobile X-ray detector is unlikely with the described method.

According to an embodiment, the predefined part of space comprises a line of sight of the X-ray tube. In this context, the line of sight of the X-ray tube may be a center line of the X-rays emitted by the X-ray tube. Hence, when the mobile X-ray detector is put in the part of space, it is in the line of sight or close to the line of sight of the X-ray tube, and hence in a part of space where it can detect the X-rays emitted by the X-ray tube. This may be particularly intuitive for staff operating the X-ray system, since a mobile detector that is placed in the area where it can detect the X-rays emitted by the X-ray tube is automatically paired with the X-ray tube system. Alternatively, or additionally, the predefined part of space comprises at least a part of an imaging area of the X-ray tube. In this context, the imaging area of the X-ray tube is the area into which the X-rays of the X-ray tube are emitted. Hence, the mobile X-ray detector may be also recognized towards the edges of the imaging area. Alternatively, or additionally, the predefined part of space is adjacent to or close to the imaging area of the X-ray tube. As an example, the predefined part of space may surround the imaging area of the X-ray tube, such that a mobile X-ray detector that is moved through the predefined part of space into the imaging area of the X-ray tube is recognized and paired with the X-ray tube system. As another example, the predefined part of space may be located on a particular side of the imaging area of the X-ray tube, such that staff operating the X-ray system may place the mobile X-ray detector in said predefined part of space to pair it with the X-ray tube system.

According to an embodiment, the at least one sensor comprises a camera. In particular, the at least one sensor may comprise a visual camera, such as a color camera, an RGB camera, and/or a black-and-white camera, and/or a depth camera. In this context, a depth camera is a camera that provides depth information, e.g., a depth map, based, for example, on a time-of-flight measurement, on triangulation and/or on structured patterns. The camera may also be an infrared or an ultraviolet camera. In particular, for performing the method, a sensor already present in the X-ray tube system may be used. That is, the visual camera and/or depth camera of the X-ray tube system may be used for the identification of the mobile X-ray detector as well as for other purposes, e.g., patient recognition, or patient placement. Hence, very efficient use of the sensor is provided. The sensor data obtained by the camera may comprise video data and/or still images.

Alternatively, or additionally, the at least one sensor comprises a radiofrequency sensor. That is, the mobile X-ray detector may be recognized using radiofrequency signals emitted by or reflected from the mobile X-ray detector.

Alternatively, or additionally, the at least one sensor comprises a sound sensor, e.g., a microphone. With said sound sensor, sounds emitted by the mobile X-ray detector may be detected. In this context, the sounds may be audible sounds, e.g., with frequencies between 16 Hz and 20 kHz, but also infrasound and/or ultrasound, e.g., with frequencies lower or higher than the audible sounds.

According to an embodiment, the at least one sensor is located in or at an X-ray tube head of the X-ray tube system. In this context, the X-ray tube head comprises the X-ray tube and may comprise further elements, such as the at least one sensor. With the at least one sensor placed in or that the X-ray tube head, the determination of a mobile X-ray detector in the predefined part of space is simplified. In particular, the at least one sensor may be oriented in the same or a similar direction as the line of sight of the X-ray tube. In particular, for a camera, this means that a line of sight of the camera, e.g., an optical axis of the lens system of the camera, points in the same or a similar direction as the line of sight of the X-ray tube. In this context, a similar direction may be a direction with less than 30°, less than 15°, or less than 5° deviation. In an example, the predefined part of space may be the part of space covered by an image area of the camera. Hence, mobile X-ray detectors in said image area of the camera are identified and paired with the X-ray tube system.

According to an embodiment, the step of identifying the mobile X-ray detector comprises recognizing a unique identifier in the sensor data. Said unique identifier uniquely identifies the mobile X-ray detector. This uniqueness may be global, i.e., each unique identifier may be issued only once in the world, or local, i.e., each unique identifier may be issued only once in a particular environment, such as a hospital. In particular, to identify the mobile X-ray detector, the unique identifier is recognized and located within the predefined part of space. Scanning for said unique identifier may be easily implemented.

According to an embodiment, the unique identifier is a machine-readable code. As an example, the machine-readable code may be a QR code or a barcode, but it may also be a serial number or a registration number. Visual codes may be permanent such that they endure clinical cleaning procedures. The machine-readable codes may also be provided as raised and depressed areas of a surface, for recognition by a depth camera. Scanning images for such machine-readable codes is well known and may be performed using existing software routines. Alternatively, or additionally, the unique identifier is a blinking pattern of a light emitting element. In this context, the light emitting element may be an LED, a laser, or a light bulb, and may also be referred to as a signaling device. The blinking pattern may comprise frequency, duration and/or color patterns of the emitted light. Yet alternatively, or additionally, the unique identifier is a coded radiofrequency signal, emitted, e.g., by a radio-frequency identification tag.

According to an embodiment, the step of identifying the mobile X-ray detector comprises recognizing the mobile X-ray detector in the sensor data, determining features of the mobile X-ray detector based on the sensor data and identifying the mobile X-ray detector based on the determined features.

Recognizing the mobile X-ray detector may be performed, e.g., using a neural network, more particularly a simple deep convolutional neural network. Alternatively, the mobile X-ray detector may be recognized using image processing, edge detection techniques and shape recognition. In particular, mobile X-ray detectors may also be recognized when they are not entirely seen in the image, e.g., when they are partly outside the image and/or when they are partly covered by other items.

Determining features of the mobile X-ray detector may also be performed using machine learning algorithms such as neural networks, or image processing techniques. For identifying the mobile X-ray detector based on the determined features, the determined features are compared to and matched with features in a list of mobile X-ray detectors, in particular of mobile X-ray detectors within a particular environment, e.g., a hospital. Besides the features, this list comprises identification information of the mobile X-ray detectors, e.g., an address in a wireless network.

In this way, easy-to-use features may be implemented to distinguish mobile X-ray detectors from one another.

According to an embodiment, the features of the mobile X-ray detector comprise a size of the mobile X-ray detector. Hence, based on this feature, X-ray detectors having different sizes can be distinguished from one another. Alternatively, or additionally, the features of the mobile X-ray detector comprise a shape of the mobile X-ray detector. In this context, shape may comprise, e.g., an aspect ratio of the mobile X-ray detector, rounded edges, or the like. Alternatively, or additionally, the features of the mobile X-ray detector comprise a model of the mobile X-ray detector, as, e.g., indicated by a model identifier. To determine these features, the image of the mobile X-ray detector may be segmented and/or classified, e.g., by a neural network, in particular a fully convolutional neural network that has been trained on real and/or synthetic images of mobile X-ray detectors. Alternatively, or additionally, the features of the mobile X-ray detector comprise at least a label on the mobile X-ray detector. Said label may be one of the unique identifiers mentioned above, i.e., a machine-readable code, a blinking pattern of a light emitting element, and/or a coded radiofrequency signal. Alternatively, or additionally, the label may be a mark, in particular a visual mark such as a painted color or a sticker. More particularly, a combination of features will uniquely identify the mobile X-ray detector, e.g., the size of the mobile X-ray detector, the shape of the mobile X-ray detector and a color of the painted color on the mobile X-ray detector. Yet alternatively, the features of the mobile X-ray detector comprise at least a signal emitted by the mobile X-ray detector, e.g., a light signal emitted by a light emitting element of the mobile X-ray detector. For example, said light signal may have a specific color or a specific blinking pattern.

According to an embodiment, between the step of recognizing the mobile X-ray detector and the step of determining features of the mobile X-ray detector, the method further comprises sending, by the second wireless communication interface, identification commands to candidate mobile X-ray detectors, and emitting, by the candidate mobile X-ray detectors, identification signals in response to the identification commands. In this context, the candidate mobile X-ray detectors may be all mobile X-ray detectors within a particular environment, e.g., within a hospital. Alternatively, the candidate mobile X-ray detectors may be determined as the mobile X-ray detectors within a vicinity of the X-ray tube system. For example, said mobile X-ray detectors may be determined by the strength of a wireless signal emitted by the mobile X-ray detectors and received by the X-ray tube system. If said strength of the wireless signal exceeds a predetermined threshold, the corresponding mobile X-ray detector is chosen as candidate mobile X-ray detector. Features of the identification signals are then part of the features of the mobile X-ray detector. In particular, among said features of the identification signals is a timing of the identification signals. For example, the identification commands are sent to the candidate mobile X-ray detectors one after another, with a time gap in between, e.g., 100 ms. Upon receipt of the identification commands, the candidate mobile X-ray detectors emit the identification signals. Hence, the mobile X-ray detectors can be distinguished from one another by the timing of their identification signals, i.e., to which identification command they respond. In this context, the identification signals may be light signals, radiofrequency signals, and/or sound signals.

According to an embodiment, identifying the mobile X-ray detector further comprises determining a location of the mobile X-ray detector and determining, based on the location of the mobile X-ray detector, whether the mobile X-ray detector is located within the predefined part of space. Said determination of the location of the mobile X-ray detector may be made by determining a position within an image. In particular, when the at least one sensor comprises a camera that is located in or at the X-ray tube head, the predefined part of space may be defined to be within the image area of said camera, or within a certain region within the image area of the camera. Alternatively, or additionally, the location of the mobile X-ray detector may be determined with a depth camera and/or via triangulation based on images, sound and/or radiofrequency signals. Only if it has been determined that the mobile X-ray detector is located within the predefined part of space, the mobile X-ray detector will be paired with the X-ray tube system.

According to an embodiment, at least a part of the step of identifying the mobile X-ray detector is performed by a neural network. In particular, said neural network may be a deep convolutional network, or a fully convolutional network. For example, the step of recognizing the mobile X-ray detector in the sensor data and/or the step of determining features of the mobile X-ray detector may be performed by a neural network. Said neural network may be trained with real data, augmented data and/or synthetic data. A properly trained neural network will provide fast and reliable results.

According to an embodiment, the method further comprises, after the step of pairing the mobile X-ray detector with the X-ray tube system, providing a confirmation signal, more particularly an audible and/or visual signal. With said confirmation signal, staff operating the X-ray system is informed that pairing of the mobile X-ray detector with the X-ray tube system has been performed and can continue with other preparations for taking the X-ray image and/or with taking the X-ray image. An audible signal may be a predefined sound and/or a spoken message. The visual signal may be a light signal emitted by the mobile X-ray detector and/or the X-ray tube system, and/or a message on a control screen of the X-ray tube system. Further, the confirmation signal may be different for different mobile X-ray detectors, e.g., having a different pitch, or a different color of the visual signal. Also, the message on the control screen of the X-ray tube system may comprise information about the paired mobile X-ray detector, e.g., its serial number.

In another aspect of the invention, an X-ray tube system is provided. Said X-ray tube system comprises an X-ray tube, at least one sensor, a computing unit, and a wireless communication interface. The X-ray tube system is configured to perform the method according to any one of claims 1 to 12. Hence, advantages and details correspond to those given in the above description of the method.

These and other aspects of the invention will be apparent from and elucidated with reference to the embodiments described hereinafter.

### BRIEF DESCRIPTION OF THE DRAWINGS

In the following, preferred embodiments of the invention will be described, by way of example only, and with reference to the drawings in which:
Fig. 1 shows a schematic side view of an X-ray tube system and a mobile X-ray detector;
Fig. 2 shows a flowchart of an embodiment of a method for pairing a mobile X-ray detector with an X-ray tube system;
Fig. 3 shows a flowchart of another embodiment of a method for pairing a mobile X-ray detector with an X-ray tube system;
Fig. 4 shows a flowchart of yet another embodiment of a method for pairing a mobile X-ray detector with an X-ray tube system; and
Fig. 5 shows a flowchart of yet another embodiment of a method for pairing a mobile X-ray detector with an X-ray tube system.

In the figures, elements which correspond to elements already described may have the same reference numerals. Examples, embodiments or optional features, whether indicated as non-limiting or not, are not to be understood as limiting the invention as claimed.

### DETAILED DESCRIPTION OF EMBODIMENTS

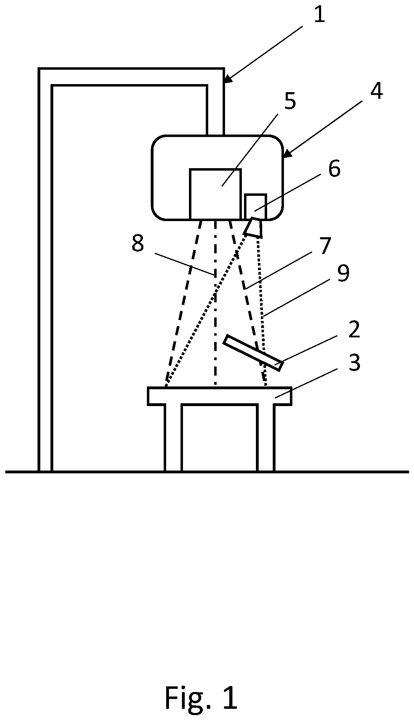

Fig. 1 shows a schematic side view of an X-ray tube system 1 and a mobile X-ray detector 2. It is understood that the mobile X-ray detector 2 will be handled by staff and be placed in a correct position to obtain an X-ray image of a patient who is not shown here and who may be placed on a patient table 3.

The X-ray tube system 1 comprises an X-ray tube head 4 with an X-ray tube 5 and a sensor 6, here shown as a camera. Said camera may be a visual camera and/or a depth camera. Other sensors, like a radiofrequency sensor and/or a sound sensor may be used instead of or in addition to the camera.

The mobile X-ray detector 2 is an X-ray detector that can be moved, in particular between different rooms. As shown here, the mobile X-ray detector 2 may be a portable X-ray detector, i.e., a mobile X-ray detector 2 that can be carried, but may also be a mobile X-ray detector 2 attached to a movable detector stand.

Further shown is an imaging area 7 of the X-ray tube 5 with a line of sight 9 of the X-ray tube 5 being at the center of the imaging area 7. Also shown is an imaging area 9 of the sensor 6.

Not shown in Fig. 1 are a first wireless communication interface of the X-ray tube system 1 and a second wireless communication interface of the mobile X-ray detector 2 as well as a computing unit of the X-ray tube system. Said first and second wireless communication interface are used for communication of the X-ray tube system 1 with the mobile X-ray detector 2.

In an alternative embodiment, a dedicated X-ray detector is provided, turning the X-ray tube system 1 into an X-ray system. In this case, staff can choose whether they want to use the dedicated X-ray detector and/or the mobile X-ray detector 2.

The X-ray tube system 1 is configured to perform a method for pairing the mobile X-ray detector 2 with the X-ray tube system 1 provided in the following Figures.

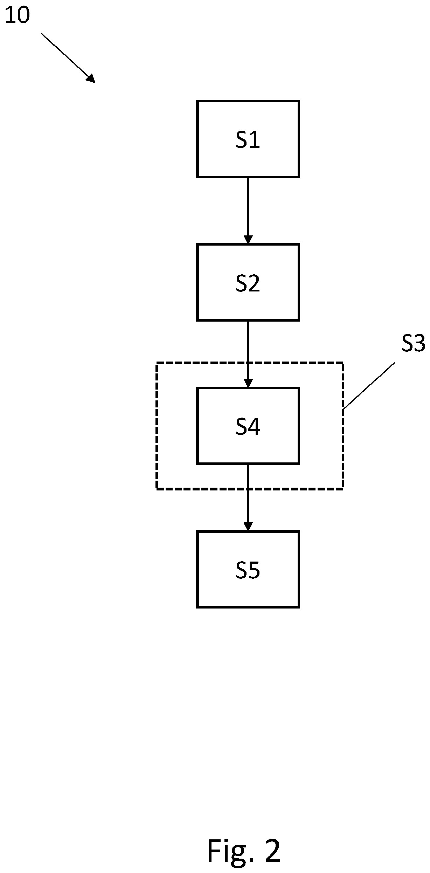

Fig. 2 shows a flowchart of an embodiment of a method 10 for pairing a mobile X-ray detector 2 with an X-ray tube system 1.

According to the method 10, sensor data is obtained S1 by at least one sensor, in particular by the sensor 6. Further, the sensor data is transmitted S2 by the at least one sensor 6 to the computing unit of the X-ray tube system 1. Accordingly, the sensor data is received by the computing unit of the X-ray tube system 1 from the at least one sensor 6. Transmitting S2 and receiving the sensor data may be performed using a wired connection and/or a wireless connection. Further, the sensor data may be obtained S1, transmitted S2 and received periodically, e.g., once per second, or several times per second. The sensor data may be raw sensor data obtained by the at least one sensor 6 or may be pre-processed by the at least one sensor 6.

Then, a mobile X-ray detector 2 located in a predefined part of space is identified S3 by the computing unit, based on the received sensor data.

The predefined part of space may comprise the line of sight 8 of the X-ray tube 5. Hence, when the mobile X-ray detector 2 is put in the part of space, it is in the line of sight 8 or close to the line of sight 8 of the X-ray tube 5, and hence in a part of space where it can detect the X-rays emitted by the X-ray tube 5. This may be particularly intuitive for staff operating the X-ray system, since a mobile X-ray detector 2 that is placed in the area where it can detect the X-rays emitted by the X-ray tube 5 is automatically paired with the X-ray tube system 1. Alternatively, or additionally, the predefined part of space may comprise at least a part of the imaging area 7 of the X-ray tube 5. In this context, the imaging area 7 of the X-ray tube 5 is the area into which the X-rays of the X-ray tube 5 are emitted. Hence, the mobile X-ray detector 2 may be also recognized towards the edges of the imaging area 7. Alternatively, or additionally, the predefined part of space may be adjacent to or close to the imaging area 7 of the X-ray tube 5. As an example, the predefined part of space may surround the imaging area 7 of the X-ray tube 5, such that a mobile X-ray detector 2 that is moved through the predefined part of space into the imaging area of the X-ray tube is recognized and paired with the X-ray tube system. As another example, the predefined part of space may be located on a particular side of the imaging area of the X-ray tube, such that staff operating the X-ray system may place the mobile X-ray detector in said predefined part of space to pair it with the X-ray tube system.

In the embodiment of Fig. 2, identifying S3 the mobile X-ray detector 2 comprises recognizing S4 a unique identifier in the sensor data. Said unique identifier uniquely identifies the mobile X-ray detector 2. This uniqueness may be global, i.e., each unique identifier may be issued only once in the world, or local, i.e., each unique identifier may be issued only once in a particular environment, such as a hospital. In particular, to identify the mobile X-ray detector 2, the unique identifier is recognized and located within the predefined part of space.

The unique identifier may be a machine-readable code. As an example, the machine-readable code may be a QR code or a barcode, but it may also be a serial number or a registration number. Visual codes may be permanent such that they endure clinical cleaning procedures. The machine-readable codes may also be provided as raised and depressed areas of a surface, for recognition by a depth camera. Scanning images for such machine-readable codes is well known and may be performed using existing software routines. Alternatively, or additionally, the unique identifier may be a blinking pattern of a light emitting element. In this context, the light emitting element may be an LED, a laser, or a light bulb, and may also be referred to as a signaling device. The blinking pattern may comprise frequency, duration and/or color patterns of the emitted light. Yet alternatively, or additionally, the unique identifier may be a coded radio-frequency signal, emitted, e.g., by a radio-frequency identification tag.

Once the mobile X-ray detector 2 that is located in the predefined part of space has been identified S3, it is paired S5 with the X-ray tube system 1. Said pairing S5 may be performed using the first wireless communication interface of the mobile X-ray detector 2 and the second wireless communication interface of the X-ray tube system 1. Pairing S5 of the mobile X-ray detector 2 with the X-ray tube system 1 may mean identifying the mobile X-ray detector 2 with the X-ray tube system 1. In particular, pairing S5 of the mobile X-ray detector 2 with the X-ray tube system 1 may comprise setting up the X-ray tube system 1 and/or the mobile X-ray detector 2 such that X-ray images may be obtained with the X-ray tube system using the mobile X-ray detector 2. More particularly, pairing S5 of the mobile X-ray detector 2 with the X-ray tube system 1 may comprise setting up a communication between the X-ray tube system 1 and the mobile X-ray detector 2, configuring the X-ray tube system 1 to acquire one or more X-ray images with the mobile X-ray detector 2, and/or configuring the mobile X-ray detector 2 to acquire one or more X-ray images with the X-ray tube system 1. This automatic pairing S5 of the mobile X-ray detector 2 with the X-ray tube system 1 significantly improves the workflow of pairing the mobile X-ray detector 2 with the X-ray tube system 1. In particular, no extra manual interaction is necessary for the pairing S5, saving time for the staff operating the X-ray system. More particularly, the workflow for operating the X-ray tube system 1 with the mobile X-ray detector 2 may be rendered more natural and fluent. Also, changing the mobile X-ray detector 2 paired with the X-ray tube system 1 is simple and fast with the described method. Moreover, no dedicated extra piece of hardware is necessary to perform the pairing S5 of the mobile X-ray detector 2 with the X-ray tube system 1. Also, the described method provides increased reliability, since pairing S5 the X-ray tube system 1 with a wrong mobile X-ray detector 2 is unlikely with the described method.

Once the mobile X-ray detector 2 has been paired with the X-ray tube system 1, a confirmation signal may be provided. Said confirmation signal may be an audible and/or a visual signal. With said confirmation signal, staff operating the X-ray system is informed that pairing of the mobile X-ray detector 2 with the X-ray tube system 1 has been performed and can continue with other preparations for taking the X-ray image and/or with taking the X-ray image. An audible signal may be a predefined sound and/or a spoken message. The visual signal may be a light signal emitted by the mobile X-ray detector 2 and/or the X-ray tube system 1, and/or a message on a control screen of the X-ray tube system 1. Further, the confirmation signal may be different for different mobile X-ray detectors 2, e.g., having a different pitch, or a different color of the visual signal. Also, the message on the control screen of the X-ray tube system 1 may comprise information about the paired mobile X-ray detector 2, e.g., its serial number.

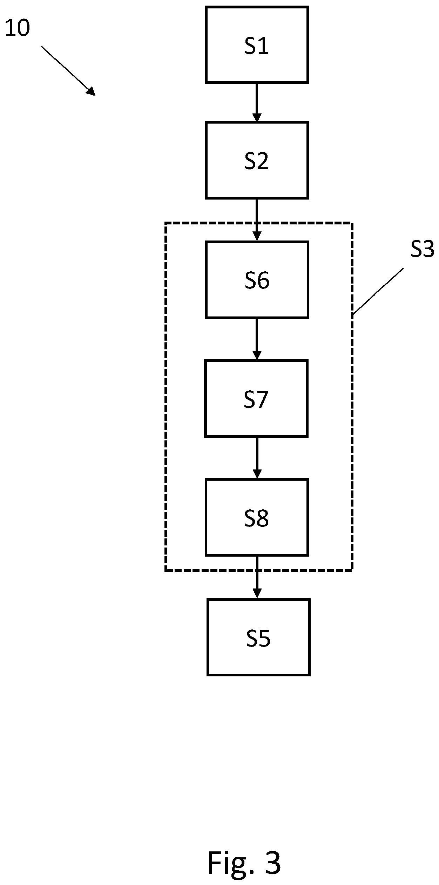

Fig. 3 shows a flowchart of another embodiment of a method 10 for pairing a mobile X-ray detector 2 with an X-ray tube system 1. In this embodiment, identifying S3 the mobile X-ray detector 2 comprises recognizing S6 the mobile X-ray detector 2 in the sensor data. Said recognizing S6 may be performed, e.g., using a neural network, more particularly a simple deep convolutional neural network. Alternatively, the mobile X-ray detector 2 may be recognized using image processing, edge detection techniques and shape recognition. In particular, mobile X-ray detectors 2 may also be recognized when they are not entirely seen in the image, e.g., when they are partly outside the image and/or when they are partly covered by other items. In particular, together with recognizing S6 the mobile X-ray detector 2, a location of the mobile X-ray detector 2 may be determined. Based on said location of the mobile X-ray detector 2, it is then determined whether the mobile X-ray detector 2 is located within the predefined part of space. Said determination of the location of the mobile X-ray detector 2 may be made by determining a position within an image. In particular, when the at least one sensor 6 comprises a camera that is located in or at the X-ray tube head 4, the predefined part of space may be defined to be within the image area of said camera, or within a certain region within the image area of the camera. Alternatively, or additionally, the location of the mobile X-ray detector 2 may be determined with a depth camera and/or via triangulation based on images, sound and/or radiofrequency signals. Only if it has been determined that the mobile X-ray detector 2 is located within the predefined part of space, the mobile X-ray detector 2 will be paired with the X-ray tube system 1.

In this embodiment, identifying S3 the mobile X-ray detector 2 further comprises determining S7 features of the mobile X-ray detector 2 based on the sensor data. Determining S7 the features of the mobile X-ray detector 2 may be performed using machine learning algorithms such as neural networks, or image processing techniques. The features of the mobile X-ray detector 2 may comprise a size of the mobile X-ray detector 2. Hence, based on this feature, mobile X-ray detectors 2 having different sizes can be distinguished from one another. Alternatively, or additionally, the features of the mobile X-ray detector 2 may comprise a shape of the mobile X-ray detector 2. In this context, shape may comprise, e.g., an aspect ratio of the mobile X-ray detector 2, rounded edges, or the like. Alternatively, or additionally, the features of the mobile X-ray detector 2 may comprise a model of the mobile X-ray detector 2, as, e.g., indicated by a model identifier. To determine these features, the image of the mobile X-ray detector 2 may be segmented and/or classified, e.g., by a neural network, in particular a fully convolutional neural network that has been trained on real and/or synthetic images of mobile X-ray detectors. Alternatively, or additionally, the features of the mobile X-ray detector 2 may comprise at least a label on the mobile X-ray detector 2. Said label may be one of the unique identifiers mentioned above, i.e., a machine-readable code, a blinking pattern of a light emitting element, and/or a coded radiofrequency signal. Alternatively, or additionally, the label may be a mark, in particular a visual mark such as a painted color or a sticker. More particularly, a combination of features may be used, e.g., the size of the mobile X-ray detector 2, the shape of the mobile X-ray detector 2 and a color of the painted color on the mobile X-ray detector 2. Yet alternatively, the features of the mobile X-ray detector 2 may comprise at least a signal emitted by the mobile X-ray detector 2, e.g., a light signal emitted by a light emitting element of the mobile X-ray detector 2. For example, said light signal may have a specific color or a specific blinking pattern.

Finally, identifying S3 the mobile X-ray detector 2 further comprises identifying S8 the mobile X-ray detector 2 based on the determined features. For this, the determined features are compared to and matched with features in a list of mobile X-ray detectors 2, in particular of mobile X-ray detectors 2 within a particular environment, e.g., a hospital. Besides the features, this list comprises identification information of the mobile X-ray detectors 2, e.g., an address in a wireless network.

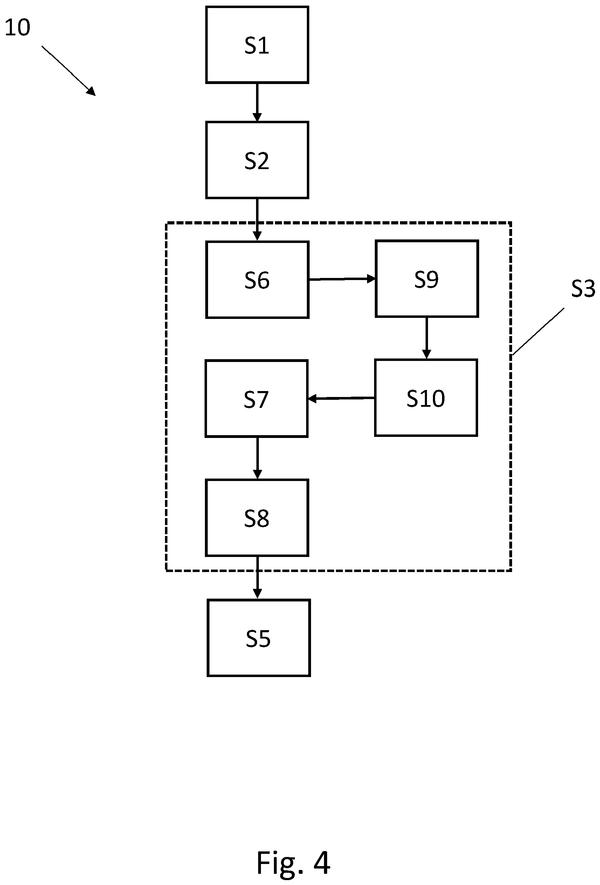

Fig. 4 shows a flowchart of yet another embodiment of a method 10 for pairing a mobile X-ray detector 2 with an X-ray tube system 1. In this embodiment, after recognizing S6 the mobile X-ray detector 2, the X-ray tube system 1 sends S9, by the second wireless communication interface, identification commands to candidate mobile X-ray detectors 2. Said candidate mobile X-ray detectors 2 may be all mobile X-ray detectors 2 within a particular environment, e.g., within a hospital. Alternatively, the candidate mobile X-ray detectors 2 may be determined as the mobile X-ray detectors 2 within a vicinity of the X-ray tube system 1. For example, said mobile X-ray detectors 2 may be determined by the strength of a wireless signal emitted by the mobile X-ray detectors 2 and received by the X-ray tube system 1. If said strength of the wireless signal exceeds a predetermined threshold, the corresponding mobile X-ray detector 2 is chosen as candidate mobile X-ray detector 2.

Once, the mobile X-ray detectors 2 have received the identification commands, they emit S10 identification signals in response to the identification commands. Features of the identification signals are then part of the features of the mobile X-ray detector 2 that are used to identify S8 the mobile X-ray detector 2. In particular, among said features of the identification signals is a timing of the identification signals. For example, the identification commands are sent to the candidate mobile X-ray detectors 2 one after another, with a time gap in between, e.g., 100 ms. Upon receipt of the identification commands, the candidate mobile X-ray detectors 2 emit the identification signals. Hence, the mobile X-ray detectors 2 can be distinguished from one another by the timing of their identification signals, i.e., to which identification command they respond. In this context, the identification signals may be light signals, radiofrequency signals, and/or sound signals.

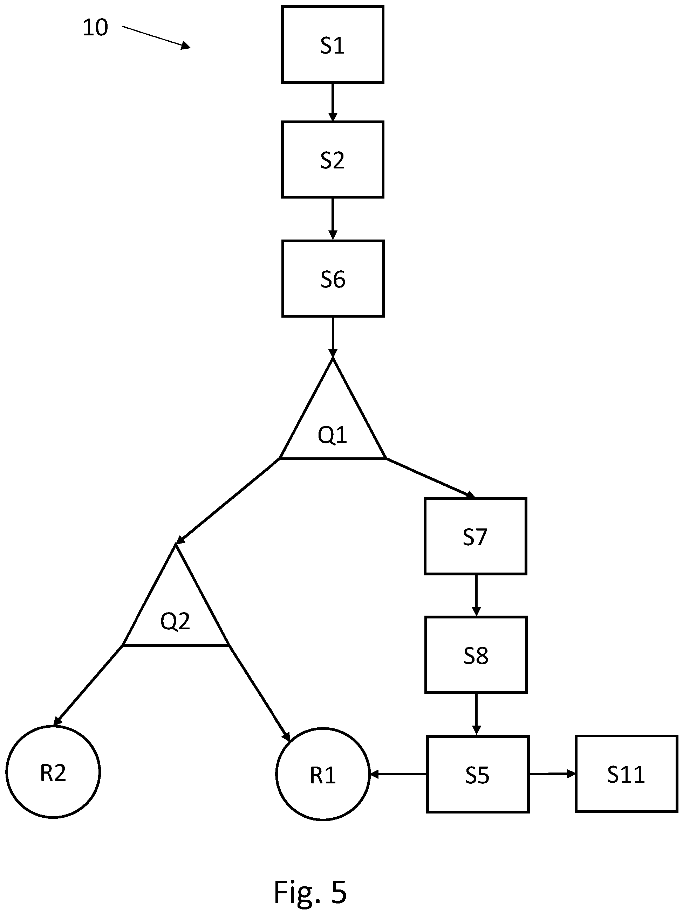

Fig. 5 shows a flowchart of yet another embodiment of a method 10 for pairing a mobile X-ray detector 2 with an X-ray tube system 1. After the step of recognizing S6 a mobile X-ray detector 2, it is queried Q1 whether a mobile X-ray detector 2 has been detected, i.e., whether a mobile X-ray detector 2 is present in the predefined part of space.

If a mobile X-ray detector 2 is present, the method 10 continues with determining S7 features of the mobile X-ray detector 2, identifying S8 the mobile X-ray detector 2 based on the determined features and pairing S5 the mobile X-ray detector 2 with the X-ray tube system 1. Once the pairing S5 has been successfully completed, a confirmation signal is provided S11 and the X-ray system is ready R1, e.g., for a medical exam.

If, on the other hand, it has been determined in the query Q1 that no mobile X-ray detector 2 is present, it is queried Q2 whether a mobile X-ray detector 2 is already paired with the X-ray tube system 1. If a mobile X-ray detector 2 is already paired with the X-ray tube system 1, the X-ray system is also ready R1. In this case, the mobile X-ray detector 2 may be, e.g., hidden by a patient, for example placed underneath the patient. If, however, no mobile X-ray detector 2 is paired with the X-ray tube system 1, the X-ray system is not ready R2.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

### LIST OF REFERENCE SIGNS:

- 1: X-ray tube system
- 2: mobile X-ray detector
- 3: patient table
- 4: X-ray tube head
- 5: X-ray tube
- 6: sensor
- 7: imaging area of the X-ray tube
- 8: line of sight of the X-ray tube
- 9: imaging area of the sensor
- 10: method

- S1: obtaining sensor data
- S2: transmitting sensor data
- S3: identifying a mobile X-ray detector
- S4: recognizing a unique identifier
- S5: pairing the mobile X-ray detector with the X-ray tube system
- S6: recognizing the mobile X-ray detector
- S7: determining features
- S8: identifying based on the determined features
- S9: sending identification commands
- S10: emitting identification signals
- S11: providing a confirmation signal

- Q1: querying whether a mobile X-ray detector has been detected
- Q2: querying whether a mobile X-ray detector is already paired

- R1: ready
- R2: not ready

## Claims

1. A method (10) for pairing a mobile X-ray detector (2) with an X-ray tube system (1), wherein the mobile X-ray detector (2) comprises a first wireless communication interface and the X-ray tube system (1) comprises an X-ray tube (5), at least one sensor (6), a computing unit and a second wireless communication interface, the method (10) comprising:
obtaining (S1), by the at least one sensor (6), sensor data;
transmitting (S2), by the at least one sensor (6), the sensor data to the computing unit;
identifying (S3), by the computing unit, based on the received sensor data, a mobile X-ray detector (2) located in a predefined part of space; and
pairing (S5), by the computing unit, via the second wireless communication interface, and via the first wireless communication interface, the mobile X-ray detector (2) with the X-ray tube system (1).

2. The method (10) according to claim 1, wherein the predefined part of space comprises a line of sight (8) of the X-ray tube (5) and/or at least a part of an imaging area (7) of the X-ray tube (5), and/or is adjacent to or close to the imaging area (7) of the X-ray tube (5).

3. The method (10) according to claim 1 or 2, wherein the at least one sensor (6) comprises a camera, more particularly a visual camera and/or a depth camera, a radiofrequency sensor, and/or a sound sensor.

4. The method (10) according to any one of claims 1 to 3, wherein the at least one sensor (6) is located in or at an X-ray tube head (4) of the X-ray tube system (1) and is, more particularly, oriented in the same or a similar direction as the line of sight (8) of the X-ray tube (5).

5. The method (10) according to any one of claims 1 to 4, wherein the step of identifying (S3) the mobile X-ray detector (2) comprises recognizing (S4) a unique identifier in the sensor data.

6. The method (10) according to claim 5, wherein the unique identifier is a machine-readable code, a blinking pattern of a light emitting element, and/or a coded radiofrequency signal.

7. The method (10) according to any one of claims 1 to 4, wherein the step of identifying (S3) the mobile X-ray detector (2) comprises:
recognizing (S6) the mobile X-ray detector (2) in the sensor data;
determining (S7) features of the mobile X-ray detector (2) based on the sensor data; and
identifying (S8) the mobile X-ray detector (2) based on the determined features.

8. The method (10) according to claim 7, wherein the features of the mobile X-ray detector (2) comprise a size of the mobile X-ray detector (2), a shape of the mobile X-ray detector (2), a model of the mobile X-ray detector (2), at least a label on the mobile X-ray detector (2) and/or at least a signal, in particular a light signal, emitted by the mobile X-ray detector (2).

9. The method (10) according to claim 7 or 8, wherein, between the step of recognizing (S6) the mobile X-ray detector (2) and the step of determining (S7) features of the mobile X-ray detector (2), the method (10) further comprises:
sending (S9), by the second wireless communication interface, identification commands to candidate mobile X-ray detectors (2); and
emitting (S10), by the candidate mobile X-ray detectors (2), identification signals in response to the identification commands,
wherein the features of the mobile X-ray detector (2) comprise features of the identification signals, more particularly a timing of the identification signals.

10. The method (10) according to any one of claims 1 to 9, wherein identifying (S3) the mobile X-ray detector (2) further comprises determining a location of the mobile X-ray detector (2) and determining, based on the location of the mobile X-ray detector (2), whether the mobile X-ray detector (2) is located within the predefined part of space.

11. The method (10) according to any one of claims 1 to 10, wherein at least a part of the step of identifying (S3) the mobile X-ray detector (2) is performed by a neural network, more particularly a deep convolutional neural network.

12. The method (10) according to any one of claims 1 to 11, further comprising, after the step of pairing (S5) the mobile X-ray detector (2) with the X-ray tube system (1):
providing (S11) a confirmation signal, more particularly an audible and/or visual signal.

13. An X-ray tube system (1), comprising
an X-ray tube (5);
at least one sensor (6);
a computing unit; and
a wireless communication interface,
wherein the X-ray tube system (1) is configured to perform the method (10) according to any one of claims 1 to 12.
